(19) Europäisches Patentamt
European Patent Office
Office européen des brevets

(11) Numéro de publication: **0 059 664**
**B1**

(12) **FASCICULE DE BREVET EUROPEEN**

(45) Date de publication du fascicule du brevet: **21.11.84**

(51) Int. Cl.³: **C 07 D 493/04** // A61K31/34, (C07D493/04, 307/00, 307/00)

(21) Numéro de dépôt: **82400328.9**

(22) Date de dépôt: **25.02.82**

(54) **Procédé de synthèse des mononitrates d'isosorbide.**

(30) Priorité: **27.02.81 FR 8103906**

(43) Date de publication de la demande: **08.09.82 Bulletin 82/36**

(45) Mention de la délivrance du brevet: **21.11.84 Bulletin 84/47**

(84) Etats contractants désignés: **AT BE CH DE FR GB IT LI LU NL SE**

(56) Documents cités:
**ORGANIC MAGNETIC RESONANCE, vol. 3, no. 6, 1971, pages 693-701 (IE) M. ANTEUNIS et al.: "Configurational and conformational insights on exo- and endo-1,4:3,6-dianhydro-glucitol mononitrate"**

(73) Titulaire: **SOCIETE NATIONALE DES POUDRES ET EXPLOSIFS, 12, quai Henri IV, F-75181 Paris Cedex 04 (FR)**

(72) Inventeur: **Emeury, Jean-Marie, Rue des Rosiers, F-84700 Sorgues (FR)**
Inventeur: **Wimmer, Eric, 2 B Chemin des Chênes, F-84700 Sorgues (FR)**

**EP 0 059 664 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

L'invention se rapporte à la synthèse des deux mononitrates d'isosorbide et à leur séparation.

L'isosorbitol ou dianhydro-1,4,3,6 sorbitol est un diol dont les groupements hydroxyles sont responsables d'une isomérie de position. On distingue ainsi deux mononitrates d'isosorbitol, génériquement dénommés MONIS dans ce qui suit, selon que c'est la fonction hydroxyle en position exo (MONIS 2) ou celle en position endo (MONIS 5) qui est nitrée:

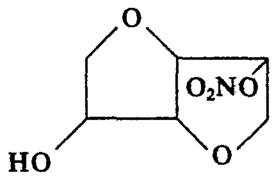

MONIS 5

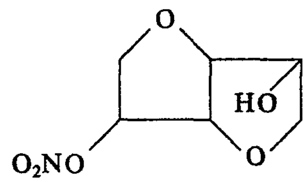

MONIS 2

Les MONIS et notamment le MONIS 2 sont connus comme vasodilatateurs coronaires à l'instar du dinitrate d'isosorbitol (DINIS) et de la nitroglycérine. Les qualités de ces composés en tant qu'intermédiaires dans le métabolisme du DINIS dans le sang ont été décrit es par R. C. Wendt, J. Pharmacol. exp. Ther. 180, 732—742 (1972), et, plus récemment dans La Nouvelle Presse Médicale, Tome 9, pages 2424 à 2427 (1980).

A ce jour, pour autant que l'on sache, il exister plusieurs procédés de synthèse des MONIS. Le plus ancien a été décrit par Csizmadia et Hayward dans Photochem. Photobiol. 4, 657 (1965), et consiste à nitrer directement l'isosorbitol. Ce procédé, qui a été repris dans le brevet américain 3 886 186, présente de graves inconvénients résidant moins dans la difficulté de séparer le mélange d'isomères obtenu (où le MONIS 2, isomère le plus intéressant n'apparait qu'à raison de 22% environ) que dans le danger potentiel recelé par l'utilisation du mélange nitrant ternaire $HNO_3$ — acide acétique — anhydride acétique. DUBAR et CALZIA, Comptes Rendus de l'Académie des Sciences de Paris, Tome 266, 1114—1116, Série C, 8 avril 1968, ont montré en effet que de tels mélanges, et notamment ceux contenant surtout de l'acide nitrique et de l'anhydride acétique, renferment du nitrate d'acétyle, considéré comme un explosif plus sensible que la nitroglycérine. MEDARD dans son ouvrage, »Les Explosifs Occasionnels«, Volume 2, p. 486—487 (1978), a plus récemment à nouveau mis en garde les utilisateurs de tels mélanges contre le danger d'explosion, d'autant plus grave de conséquences que le mélange nitrant suspect est présent en plus grande proportion dans le mélange réactionnel.

Un autre procédé a été proposé, qui est décrit dans le brevet américain 4 065 488. Ce procédé consiste à former un mélange de monoacétates et de diacétates d'isosorbitol dans un premier temps, puis à nitrer ce mélange à l'aide d'un mélange d'acide nitrique concentré et d'anhydride acétique et enfin à hydrolyser le mélange obtenu et à isoler le MONIS 2. Ce procédé a l'avantage de conduire à un mélange d'isomères où le MONIS 2 est majoritaire par rapport au MONIS 5 mais cet avantage est compensé par de sérieux inconvénients: rendement relativement faible, temps de réaction très long (2 à 5 jours), grand nombre d'étapes et, surtout, utilisation d'un mélange nitrant potentiellement explosif, notamment en cas d'erreur de dosage.

Malgré ses inconvénients importants, ce dernier procédé apparaît jusqu'à présent comme le plus sûr, car, fait-on valoir, il n'implique la formation que faibles quantités de DINIS, qui est un explosif connu.

Ainsi on ne dispose pas, à l'heure actuelle, d'un procédé sûr et aussi léger que possible, conduisant à de bons rendements en MONIS 2.

La Demanderesse a maintenant trouvé un procédé permettant de résoudre le problème posé.

L'invention consiste en un procédé de synthèse de mononitrates d'isosorbide caractérisé en ce qu'on dénitre le dinitrate d'isosorbide au moyen de l'hydrazine on d'un dérivé hydrazinique dans un milieu solvant polaire.

Il a en effet été trouvé que, contre toute attente, il est possible, dans certaines conditions, d'éliminer tout danger d'explosion dans la manipulation du DINIS, contrairement aux idées reçues, de décomposer le DINIS en MONIS sans que la décomposition atteigne le stade de l'isosorbitol et d'orienter la dénitration vers la formation préférentielle de MONIS 2. Ceci est largement surprenant car la mise en œuvre d'autres moyens d'hydrolyse partielle tels que la pyridine ou l'acide sulfurique conduit précisément à ce à quoi on peut normalement s'attendre, à savoir à des mélanges de DINIS, de MONIS et d'isosorbitol et, dans les MONIS obtenus, à une proportion élevée de MONIS 5 étant donné que cet isomère est celui qui résulte de l'hydrolyse de celui des groupes nitrates du DINIS qui est le plus accessible.

Les conditions mises en évidence selon l'invention sont essentiellement celles qui viennent d'être citées à savoir l'emploi de l'hydrazine ou d'un dérivé hydrazinique et d'un milieu solvant polaire.

Par dérivé hydrazinique, on entend les composés qui dérivent de l'hydrazine par substitution d'au moins un et d'au plus trois des atomes d'hydrogène qu'elle porte sur ses atomes d'azote, par des

groupes hydrocarbonés tels que des groupes aliphatiques renfermant de 1 à 10 atomes de carbone, des groupes aromatiques renfermant de 6 à 12 atomes de carbone, éventuellement substitués, notamment par des atomes d'halogène, des groupes amino ou des groupes nitro , des groupes aliphato-aromatiques tel que le groupe benzyle et des groupes acyle tel que le groupe acétyle ou benzoyle et les composés d'addition des composés précédents.

D'une manière générale on préfère utiliser dans le cadre de la présente invention un dérivé hydrazinique de type connu pour être un réducteur normalement efficace et, à cet égard, l'invention comprend également l'utilisation de composés d'addition des hydrazines, notamment les hydrates et les chlorhydrates. Ainsi on utilise volontiers l'hydrazine, l'hydrate d'hydrazine, ainsi que les hydrazines mono ou di-substituées telles que la monométhylhydrazine, la N,N'-diméthylhydrazine, la N,N-diméthylhydrazine, la monophénylhydrazine et leurs hydrates, et de préférence, d'une manière générale, celles des hydrazines qui donne lieu à la formation de la plus faible quantité possible de produits susceptibles de diminuer la polarité du milieu réactonnel, après réaction sur le DINIS. Dans cet ordre d'idées, on évite aussi, de préférence, d'utiliser des dérivés hydraziniques qui, après réaction, donnent lieu à la formation de composés solides ou liquides susceptibles de former une phase supplémentaire dans le milieu réactionnel, qui comprend, comme il a été dit un milieu solvant polaire.

Il a été trouvé de manière surprenante que les hydrazines mono ou di-substituées et leurs composés d'addition et notamment les di-substitués comme le N,N-diméthyl hydrazine permettaient d'obtenir des rendements supérieurs.

Par milieu solvant polaire, on doit entendre un milieu présentant au moins une phase renfermant un solvant ou un mélange de solvants, qui présente à la fois une bonne solubilité à l'égard du DINIS et du dérivé hydrazinique et une constante diélectrique supérieure à 3 à 20° C. Conformément à l'invention, on peut donc utiliser un mélange hétérogène de solvants, dont l'une des phases seulement possède la condition de polarité indiquée plus haut. Un milieu constitué d'une seule phase convient toutefois bien. On apprécie notamment un mélange de solvants polaires, de proticité différente, par exemple l'un étant protique et l'autre non.

Des exemples de tels mélanges sont constitués par des mélanges de composés aromatiques et d'alcools aliphatiques comme les mélanges toluène-éthanol, toluène-propanol, toluène-méthanol, benzène-éthanol, benzène-méthanol, benzène-propanol ou des mélanges d'éthers et d'alcools aliphatiques comme les mélanges THF-éthanol, dioxanne-méthanol, dioxanne-éthanol, THF-méthanol, ces deux derniers mélanges étant particulièrement préférés.

Le DINIS utilisé dans le cadre de la présente invention comme produit de départ est un composé explosif dont les caractéristiques détoniques sont bien connues. La Demanderesse a pu se rendre compte que la manipulation du DINIS ne posait aucune difficulté du point de vue de la sécurité dès le moment où ce produit est humide ou imprégné de solvant ou en solution, de sorte qu'il n'est pas forcément utile d'isoler le DINIS qui vient d'être synthétisé avant de le traiter par l'hydrazine ou un dérivé hydrazinique conformément à l'invention.

En principe il est possible d'utiliser la proportion stoechiométrique de l'hydrazine ou de dérivé hydrazinique par rapport au DINIS. En pratique on utilise de 1 à 2 moles ou plus par mole de DINIS, de préférence de 1,1 à 1,5 moles. Naturellement cette proportion molaire doit s'apprécier en nombre de fonctions hydraziniques efficaces portées par le dérivé hydrazinique, si ce dernier est un composé polyhydrazinique.

La température régnant dans le milieu réactionnel n'est pas un facteur très critique mais il est préférable pour obtenir une durée de réaction industriellement satisfaisante, d'opérer la réaction selon l'invention à une température supérieure à 40° C, de préférence supérieure à 60° C, mais inférieure et de préférence égale à la température de reflux du milieu solvant utilisé qui est généralement de l'ordre de 65° C, et ne doit pas, en tout état de cause, dépasser 150° C.

Naturellement on ne sort pas du cadre de la présente invention en utilisant des catalyseurs de l'hydrolyse par l'hydrazine ou les dérives hydraziniques. Comme tels catalyseurs on peut citer, par exemple, le palladium, le platine ainsi que des bases fortes telles que l'éthoxyde de sodium. Toutefois la réaction est généralement suffisamment rapide en l'absence de catalyseur, pour qu'on puisse aisément se passer de ces derniers.

L'ordre d'introduction des réactifs n'est pas un facteur critique dans le procédé selon l'invention. Il est toutefois préférable de constituer d'abord une solution du DINIS dans le milieu solvant polaire, et seulement ensuite d'introduire l'hydrazine ou le dérivé hydrazinique pur ou en solution. L'introduction est une opération qui peut être réalisée en l'espace de quelques minutes, par exemple en 15 minutes, ce qui est avantageux tant du point de vue du temps gagné que de la qualité des produits obtenus. L'introduction étant achevée, le milieu réactionnel ne nécessite pas d'autres soins. On peut éventuellement maintenir agité le milieu réactionnel, ce qui peut être obtenu soit mécaniquement, soit en opérant à reflux du solvant ce qui, comme il a été dit plus haut, apparaît comme la solution la plus avantageuse. La réaction peut être considérée comme achevée au bout de 1 à 10 heures suivant les conditions et plus généralement au bout de 2 à 7 heures, ce qui autorise une application du procédé en continu, si on le désire. On chasse alors le solvant, par exemple sous vide, on filtre le cas échéant le milieu réactionnel pour éliminer le DINIS qui éventuellement n'a pas réagi et qui a alors précipité. Le résidu contient les MONIS à l'exclusion de toutes autres quantités appréciables de sous-produits. Le

résidu est repris par de l'eau et la phase aqueuse ainsi obtenue est extraite à l'aide d'un solvant organique des MONIS, tel qu'un hydrocarbure aliphatique chloré. Après évaporation du solvant, on obtient une huile de couleur jaune constituée de MONIS 2 et de MONIS 5 que l'on sépare par tout procédé classique de séparation et notamment par cristallisation fractionnée ou par chromatographie. Cette dernière méthode peut être appliquée avantageusement dans les colonnes de grands diamètres à remplissage de Silice, le solvant d'élution étant avantageusement le couple $CCl_4$/acetone dans le rapport 4 : 1.

On obtient ainsi environ deux fois plus de MONIS 2 que de MONIS 5, le rendement global de la réaction variant selon les conditions retenues entre 15 et 60% en poids, en MONIS 2, par rapport au DINIS de départ. Le MONIS 2 obtenu dans les conditions de l'invention a une pureté d'emblée intéressante puisqu'il présente un point de fusion compris entre 52,5 et 53° C au sortir de la séparation et un point de fusion supérieur ou égal à 54° C après une première recristallisation effectuée sur le produit isolé.

Le procédé selon l'invention conduisant à un mélange ne contenant que les deux isomères recherchés, on recueille aussi facilement le MONIS 5 directement sous forme d'un solide blanc fondant à 89° C, ce qui est conforme aux indications de la littérature pour le produit pur.

L'invention sera mieux comprise à l'aide de l'exemple suivant qui illustre, sans la limiter, la présente invention:

## Exemple 1

Dans un réacteur de Keller de 500 ml muni d'un agitateur, d'un réfrigérant à reflux et d'un dispositif de coulée on mélange 150 ml de THF, 150 ml de méthanol et 59,0 g (0,25 mole) de DINIS.

L'agitation étant mise en marche, on porte le mélange à reflux.

La reflux étant atteint, on coule dans le milieu 17,5 g (0,35 mole) (soit un excès molaire de 40% par rapport au DINIS) d'hydrate d'hydrazine, en 15 minutes. L'introduction une fois achevée, on maintient le reflux pendant 3 heures. On arrête le chauffage, on évapore les solvants sous vide et on filtre le précipité formé, qui est constitué par du DINIS réutilisable. La phase liquide est reprise par 150 ml d'eau, puis extraite par quatre fois 50 ml de chloroforme. Les phases organiques sont combinées et on évapore le chloroforme sous vide: on obtient une huile jaune qui se fige lentement et qui apparait comme étant constituée uniquement de MONIS 2 et de MONIS 5.

On a séparé les deux isomères sur une colonne de 45 mm de diamètre intérieur et de 50 cm de hauteur, remplie de gel de silice Merck de granulométrie 0,063 à 0,2 mm (70—230 Mesh ASTM), le solvant d'élution étant un mélange 80/20 de tétrachlorure de carbone et d'acétone.

On a obtenu d'une part 13,4 g de MONIS 2, soit un rendement global de 28% et d'autre part 6,5 g de MONIS 5.

Les produits avaient les caractéristiques spectrales et physiques suivantes:

| | Spectre Infra Rouge<br>(a) OH | (b) $ONO_2$ | (b) COC |
|---|---|---|---|
| MONIS 2 | 3553 $cm^{-1}$ | 1643, 1272 et 845 $cm^{-1}$ | 1084 $cm^{-1}$ |
| MONIS 5 | 3620 $cm^{-1}$ | 1645, 1280 et 845 $cm^{-1}$ | 1092 $cm^{-1}$ |

(a) Solutions à 0,1% dans $CCl_4$, cellules 1 cm en Infrasil.
(b) Solutions à 1% dans le benzène, cellules NaCl de 100 µm.

| | Propriétés physiques<br>Température de fusion (° C) | Pouvoir rotatoire (c) | ATD (d) |
|---|---|---|---|
| MONIS 2 | 54,0 | 71,0° | 57° —168° C |
| MONIS 5 | 88,9 | 173,5° | 94° —173° C |

(c) Valeur de $\alpha$ , pour une solution à 2% dans l'acétate d'éthyle.
(d) Analyse Thermique Différentielle:
la première valeur indiquée correspond à un pic endothermique (fusion) et la seconde à un pic exothermique (décomposition).

Exemple 2

En suivant le mode opératoire de l'exemple 1 on mélange 300 ml de dioxanne, 300 ml de méthanol et 118,0 g (0,5 mole) de DINIS. Le reflux étant atteint, on coule 35,0 g (0,7 mole) d'hydrate d'hydrazine, en 30 minutes. Le reflux est maintenu pendant 6 h. Après évaporation des solvants sous vide, filtrage du DINIS et purification on obtient le mélange de MONIS 2 et 5 avec un rendement de 51%. La proportion des deux isomères est identique à celle de l'exemple 1.

Exemple 3

En suivant le mode opératoire de l'exemple 1, on mélange 300 ml de dioxanne, 300 ml d'éthanol et 118,0 g (0,5 mole) de DINIS. Le reflux étant atteint, on coule 35,0 g (0,7 mole) d'hydrate d'hydrazine, en 1 h. Le reflux est maintenu pendant 5 h. Après évaporation des solvants sous vide, filtrage du DINIS et purification on obtient le mélange de MONIS 2, et 5, dans les mêmes proportions que précédemment, avec un rendement de 50%.

Exemple 4

Deux essais ont été effectués avec un mélange toluène-éthanol dans des proportions différentes:

a) toluène 70 — éthanol 30 (en ml)
b) toluène 30 — éthanol 70

Le mode opératoire est identique aux précédents. C'est à dire que l'on utilise les mêmes proportions de DINIS et d'hydrate d'hydrazine dans 600 ml de solvant.

La coulée est effectuée en 1 h et le reflux est maintenu 5 h. Après les opérations d'évaporation, de filtrage et de purification on obtient le mélange de MONIS 2 et 5, dans les mêmes proportions que précédemment, avec respectivement les rendements suivants:

— 19% pour le solvant a)
— 38% pour le solvant b)

Exemple 5

Un essai identique à l'exemple 3 a été effectué en remplaçant l'hydrate d'hydrazine par l'hydrate de N,N-diméthylhydrazine.

On mélange 300 ml de dioxanne, 300 ml d'éthanol et 118,0 g (0,5 mole) de DINIS. Le reflux étant atteint, on coule 35,0 g (0,7 mole) d'hydrate de N,N-dimethylhydrazine en solution dans un mélange de 10 ml d'éthanol et 10 ml de dioxanne. Après évaporation, filtrage, purification on obtient un mélange de MONIS 2 et 5 dans les mêmes proportions que précédemment, avec un rendement de 80%.

Exemple 6

En suivant le mode opératoire de l'exemple 1, mélange 600 ml d'éthanol et 118,0 g (0,5 mole) de DINIS. Le reflux étant atteint, on coule 35,0 g (0,7 mole) d'hydrate d'hydrazine en 1 h. Le reflux est maintenu pendant 5 h. Après évaporation des solvants sous vide, filtrage du DINIS et purification, on obtient le mélange de MONIS 2 et 5 dans les mêmes proportions que précédemment avec un rendement de 38%.

**Revendications**

1. Procédé de synthèse de mononitrates d'isosorbide caractérisé en ce qu'on dénitre le dinitrate d'isosorbide dans un milieu solvant polaire au moyen de l'hydrazine ou d'un dérivé hydrazinique qui est choisi dans le groupe constitué par les composés dérivés de l'hydrazine par substitution d'au moins un et d'au plus trois des atomes d'hydrogène qu'elle porte sur ses atomes d'azote, par des groupes hydrocarbonés tels que des groupes aliphatiques renfermant de 1 à 10 atomes de carbone, des groupes aromatiques renfermant de 6 à 12 atomes de carbone, éventuellement substitués, notamment par des atomes d'halogène, des groupes amino ou des groupes nitro, des groupes aliphato-aromatiques tel que le groupe benzyle et des groupes acyle tel que le groupe acétyle ou benzoyle et par les composés d'addition des composés précédents, tels qu'un hydrate ou un chlorhydrate.

2. Procédé selon la revendication 1, caractérisé en ce que le milieu solvant polaire est un milieu présentant au moins une phase renfermant un solvant ou un mélange de solvants qui présente à la fois une bonne solubilité à l'égard du dinitrate d'isosorbide et de l'hydrazine ou du dérivé hydrazinique et une constante diélectrique supérieure à 3 à 20° C.

3. Procédé selon la revendication 2, caractérisé en ce que l'on utilise un mélange de solvants de proticités différentes.

4. Procédé selon la revendication 3, caractérisé en ce que le mélange est choisi dans le groupe constitué par les mélanges THF-éthanol, dioxanne-méthanol, dioxanne-éthanol, THF-méthanol, ces deux derniers mélanges étant préféreés.

5. Procédé selon l'une quelconque des revendications précédentes, caractérisé en ce que l'on utilise de 1 à 2 moles d'hydrazine ou de dérivé hydrazinique par mole de dinitrate d'isosorbide, de préférence de 1,1 à 1,5 moles.

6. Procédé selon l'une quelconque des revendications précédentes, caractérisé en ce que l'on opère la réaction à une température supérieure à 40° C, de préférence supérieure à 60° C, mais inférieure et de préférence égale à la température de reflux du milieu solvant utilisé.

7. Procédé selon l'une quelconque des revendications précédentes, caractérisé en ce que l'on utilise un catalyseur de l'hydrolyse par l'hydrazine ou les dérivés hydraziniques.

8. Procédé selon l'une quelconque des revendications précédentes, caractérisé en ce que l'on constitue d'abord une solution du dinitrate d'isosorbide dans le milieu solvant polaire, puis on introduit l'hydrazine ou le dérivé hydrazinique sur ledit mélange, porté à température de reflux du milieu solvant.

9. Procédé selon la revendication 8, caractérisé en ce que l'on introduit l'hydrazine ou le dérivé hydrazinique en environ 15 minutes.

**Patentansprüche**

1. Verfahren zur Herstellung von Isosorbidmononitraten, dadurch gekennzeichnet, daß man Isosorbiddinitrat in einem polaren Lösungsmittel-Medium mit Hydrazin oder einem Hydrazinderivat denitriert, ausgewählt aus der Gruppe bestehend aus Derivaten des Hydrazins, entstanden durch Substitution von mindestens einem und höchstens drei Wasserstoffatomen an den Stickstoffatomen durch Kohlenwasserstoffgruppen, wie aliphatischen Gruppen mit 1 bis 10 Kohlenstoffatomen, aromatischen Gruppen mit 6 bis 12 Kohlenstoffatomen, die gegebenenfalls substituiert sind, insbesondere mit Halogenatomen, Amino- oder Nitrogruppen, aliphatisch-aromatischen Gruppen, wie die Benzylgruppe, und Acylgruppen, wie die Acetyl- oder Benzoylgruppe, sowie Additionsverbindungen dieser vorstehenden Verbindungen, wie einem Hydrat oder Chlorhydrat.

2. Verfahren nach Anspruch 1, dadurch gekennzeichnet, daß das polare Lösungsmittel-Medium mindestens eine ein Lösungsmittel oder ein Gemisch von Lösungsmitteln enthaltende Phase hat, die zugleich eine gute Löslichkeit für das Isosorbiddinitrat und das Hydrazin oder Hydrazinderivat und eine Dielektrizitätskonstante über 3 bei 20° C aufweist.

3. Verfahren nach Anspruch 2, dadurch gekennzeichnet, daß man ein Gemisch von Lösungsmitteln mit unterschiedlichen protischen Eigenschaften verwendet.

4. Verfahren nach Anspruch 3, dadurch gekennzeichnet, daß das Gemisch aus der aus Gemischen von THF-Ethanol, Dioxan-Methanol, Dioxan-Ethanol, THF-Methanol bestehenden Gruppe ausgewählt wird, wobei die beiden letzteren Gemische bevorzugt sind.

5. Verfahren nach einem der vorstehenden Ansprüche, dadurch gekennzeichnet, daß man 1 bis 2 Mol Hydrazin oder Hydrazinderivat je Mol Isosorbiddinitrat, vorzugsweise 1,1 bis 1,5 Mol, verwendet.

6. Verfahren nach einem der vorstehenden Ansprüche, dadurch gekennzeichnet, daß man die Reaktion bei einer Temperatur über 40° C, vorzugsweise über 60° C , aber unterhalb und vorzugsweise bei der Rückflußtemperatur des verwendeten Lösungsmittel-Mediums durchführt.

7. Verfahren nach einem der vorstehenden Ansprüche, dadurch gekennzeichnet, daß man einen Katalysator für die Hydrolyse mit Hydrazin oder den Hydrazinderivaten verwendet.

8. Verfahren nach einem der vorstehenden Ansprüche, dadurch gekennzeichnet, daß man zuerst eine Lösung des Isosorbiddinitrats in dem polaren Lösungsmittel-Medium herstellt, dann dem auf die Rückflußtemperatur des Lösungsmittel-Medium gebrachten genannten Gemisch das Hydrazin oder Hydrazinderivat zugibt.

9. Verfahren nach Anspruch 8, dadurch gekennzeichnet, daß man das Hydrazin oder Hydrazyinderivat in etwa 15 min zugibt.

**Claims**

1. Process for the synthesis of isosorbide mononitrates, characterised in that isosorbide dinitrate is denitrated in a polar solvent medium by means of hydrazine or a hydrazine derivative which is chosen from the group consisting of compounds derived from hydrazine by substitution of at least one and at

most three of the hydrogen atoms which it carries on its nitrogen atoms, by hydrocarbon groups such as aliphatic groups containing from 1 to 10 carbon atoms, aromatic groups containing from 6 to 12 carbon atoms, which are optionally substituted, in particular by halogen atoms, amino groups or nitro groups, aliphatic-aromatic groups such as the benzyl group, and acyl groups such as the acetyl or benzoyl group, and addition compounds of the above compounds, such as a hydrate or a hydrochloride.

2. Process according to Claim 1, characterised in that the polar solvent medium is a medium having at least one phase containing a solvent or a mixture of solvents which has both a good solubility with respect to the isosorbide dinitrate and the hydrazine or hydrazine derivative, and a dielectric constant of more than 3 at 20° C.

3. Process according to Claim 2, characterised in that a mixture of solvents of different proticities is used.

4. Process according to Claim 3, characterised in that the mixture is chosen from the group consisting of THF-ethanol, dioxane-methanol, dioxane-ethanol and THF-methanol mixtures, these last two mixtures being preferred.

5. Process according to any one of the preceding Claims, characterised in that from 1 to 2 moles of hydrazine or hydrazine derivative are used per mole of isosorbide dinitrate, preferably from 1.1 to 1.5 moles.

6. Process according to any one of the preceding claims, characterised in that the reaction is carried out at a temperature above 40°, preferably above 60° C, but below and preferably at the reflux temperature of the solvent medium used.

7. Process according to any one of the preceding claims, characterised in that a catalyst is used for the hydrolysis by the hydrazine or hydrazine derivatives.

8. Process according to any one of the preceding claims, characterised in that a solution of the isosorbide dinitrate in the polar solvent medium is initially made up and the hydrazine or hydrazine derivative is then introduced into the said mixture, heated to the reflux temperature of the solvent medium.

9. Process according to Claim 8, characterised in that the hydrazine or hydrazine derivative is introduced in the course of about 15 minutes.